Europäisches Patentamt

European Patent Office

Office européen des brevets

(19)

(11) Publication number: **0 076 421**

**A2**

(12) **EUROPEAN PATENT APPLICATION**

(21) Application number: **82108672.5**

(22) Date of filing: **20.09.82**

(51) Int. Cl.³: **A 61 M 1/03**
**B 01 D 13/00**

(30) Priority: **05.10.81 SE 8105850**

(43) Date of publication of application:
**13.04.83 Bulletin 83/15**

(84) Designated Contracting States:
**AT BE CH DE FR GB IT LI LU NL**

(71) Applicant: **GAMBRO AB**
**Box 10101**
**S-220 10 Lund(SE)**

(72) Inventor: **Christopherson, Kjell**
**Tamburinvägen 10**
**S-245 00 Staffanstorp(SE)**

(72) Inventor: **Falkvall, Thore**
**Karl X Gustafs gata 67**
**S-252 40 Helsingborg(SE)**

(72) Inventor: **Mattisson, Ulf**
**Ollonborrevägen 11**
**S-240 17 S. Sandby(SE)**

(72) Inventor: **Shaldon, Stanley**
**Villa 86 Rue de Grezac**
**Montpellier(FR)**

(74) Representative: **Boberg, Nils Gunnar Erik**
**HOLGER CRAFOORD AB Patent Department Box 10101**
**S-220 10 Lund(SE)**

(54) **Filtering device.**

(57) A filtering device comprising an inlet (8) and an outlet (9) for the liquid filtered and an outlet (10) for the filtrate, the filtration being arranged to take place in two chambers (2,3) connected in series, which consist at least partly of membrane material through which the filtration is taking place.

The device is intended primarily to be used in connection with the medical treatment of blood and here in particular in connection with haemofiltration or plasmafers, in which methods a thickening of the blood and consequently a pressure drop occurs.

The characteristic feature of the invention is a pressure-controlled overflow valve (10) arranged between the inlet (8) and the outlet (9) for the liquid filtered.

Fig.1

## TITLE

FILTERING DEVICE

## TECHNICAL FIELD

The present invention relates to a filtering device comprising an inlet and an outlet for the liquid filtered and an outlet for the filtrate, the filtration being arranged to take place in two chambers connected in series, which consist at least partly of membrane material through which the filtration is taking place.

The invention is intended primarily to be used in connection with medical treatment of blood, and here in particular in connection with haemofiltration or plasmafers, in which methods a thickening of the blood and consequently a pressure drop occurs.

Haemofiltration thus refers to a process wherein the blood fluid is withdrawn from the patient together with toxins which could not be removed by means of the normal renal function. Since a quantity of liquid in the order of magnitude of 20 litres is withdrawn from the patient, substantially the same quantity of replacement liquid is supplied, insofar as a certain reduction in weight is not desired.

Plasmafers refer to a process wherein only the blood plasma is withdrawn from the blood donor and the blood cells and similar larger molecules together with a small portion of the blood plasma are returned. In this process too a certain quantity of replacement liquid may be supplied to the patient.

Both, haemofiltration and plasmafers, take place appropriately with the help of membrane filtration in devices which resemble normal membrane dialysers. Usually, however, more permeable membranes and higher pressures are used. Moreover, of course, no dialysis liquid is to be found on the side of the membrane remote from the blood.

## BACKGROUND ART

Normally separate filtering devices are used for processes of the abovementioned type which are thus similar to normal membrane dialysers. Tests have also been carried out with filtering devices coupled in parallel or in series. Especially suitable is the connection in series when an increase in pressure drop is obtained which in turn gives an increase in the amount of filtrate. However, if the pressure drop across the filtering device is too great, problems may arise. For example
the blood may be damaged through haemolysis.

Experiments have also been carried out with chambers connected
in series and in parallel arranged in one and the same casing. See
for example US patent specification 4 038 190. Difficulties may
also be experienced, however, with the designs described in this
patent if, in particular for haemofiltration or plasmafers, a sufficiently high pressure is to be achieved without the risk of too
high a pressure when, for example, the filter becomes choked.

DISCLOSURE OF INVENTION.

The abovementioned problem is solved in accordance with the
invention with the help of a filtering device comprising an inlet
and an outlet for the liquid filtered and an outlet for the filtrate,
the filtration being arranged to take place in two chambers connected
in series, which consist at least partly of membrane material through
which the filtration is taking place.

The characteristic feature of the invention is a pressure controlled
overflow valve arranged between the inlet and the outlet for the
liquid filtered.

The filtering device in accordance with the invention may be
of the so-called plane film type or it may consist of a membrane tube
wound into a spiral. In a preferred embodiment, however, it is of
the fibrous type, both chambers being arranged in one and the same
casing and each being formed of a number of interior spaces of
thin-walled fibres. In filtering devices of this type a relatively
high pressure can be used whilst the risk of a leakage through membrane
ruptures remains small.

The fibres of the two chambers may be cast in a conventional
manner into two end walls of a cylindrical casing which is provided,
outside these end walls, with outer covers bearing the said inlet
and outlet for the filtered liquid. It has been found suitable in
this connection to arrange the said inlet and outlet in one of the
outer covers, separated by a seal arranged between the adjoining end
wall and the cover, which constitutes the said pressure-controlled
overflow valve. This principle permits different simple design
solutions for the flow valve.

-3-                    **0076421**

It may be ring-shaped, for example, and arranged sub-
stantially concentrically on the inside of the  outer cover,
pressing against the outside of the adjoining end wall.  The
fibres cast into the end walls, which thus open  out on the  outside of these walls, are divided in this manner into two groups,
each of them forming the aforementioned two chambers.

To prevent the seal from stopping up some of the fibres,
it is appropriate to cast a prop ring in the end wall in front of
the seal.

A simple design is achieved by  the seal being constituted of
an-elastic ring, slipped  onto a rigid flange fixed on the inside
of the outer cover.  This rigid flange may be interrupted by one or
more ports which are only partly covered on their one side by the
elastic ring, which, however, wholly but resiliently covers the
other side of the ports.  The elastic ring is preferably given a
U-shaped cross-section with one leg longer than the other.

Alternatively the seal may be formed by a fixed ring arranged
between the outer cover and the adjoining end wall, provided with
one or more radial ports or windows, the outside of which is covered
by an elastic ring slipped onto the fixed ring.  This elastic ring
may be arranged in a peripheral groove in the fixed ring.  By  a
suitable choice of the size or rather elasticity of the elastic
ring, it is possible for the said ports or windows to open at a certain
maximum pressure, which thus cannot be exceeded.

An elastic sealing ring is appropriately arranged between the
outer cover and the fixed ring which in turn may be glued, or be
attached in some other manner, to the adjoining end wall or the
prop ring cast into it.

In accordance with a further alternative the seal may be formed
by an elastic ring, arranged between the outer cover and the adjoining
end wall, which comprises thinner wall portions which tightly adhere
to one another at lower pressure, but which are adapted so as to be
separated at higher pressure.

BRIEF DESCRIPTION OF DRAWINGS

The invention will be described in more detail in the following with reference to the enclosed drawings, which by way of example show a number of different preferred embodiments of the same.

Fig.1 shows a first embodiment of the filtering device in accordance with the invention.

Fig.2 shows an enlarged detail from fig.1.

Fig.3 shows a second embodiment of the subject of the invention.

Fig.4 shows an enlarged detail from fig.3.

Fig.5 shows a third embodiment of the subject of the invention.

Fig.6 shows an enlarged detail from fig.5.

Fig.7 shows a fourth embodiment of the subject of the invention.

Figures 8 and 9 finally show a section and an end elevation respectively of a detail from fig.7 on an enlarged scale.


BEST MODE OF CARRYING OUT THE INVENTION

EMBODIMENT I

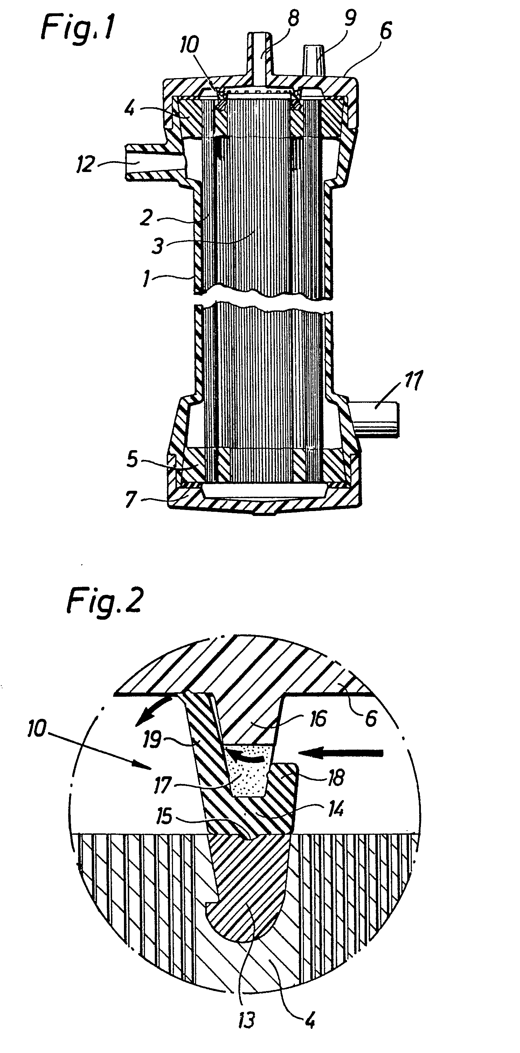

In fig.1 is shown as an example a filtering device in accordance with the invention comprising a casing or a housing 1 with a great number of fibres arranged as two concentric bundles 2 and 3 respectively. These fibres are cast into two end walls 4 and 5 respectively and open out on the outsides of these end walls. Outside the end walls 4 and 5 outer covers 6 and 7 respectively are attached to the casing 1. The outer cover 6 is provided with an inlet 8 and and outlet 9 for the liquid filtered. Between these is arranged a pressure-controlled overflow valve 10. The design of this valve is shown on an enlarged scale in fig.2. The casing 1 is provided,moreover, with an outlet 11 for the filtrate and,for reasons connected with manufacture,with an inlet 12 which may be used, for example, for the scavenging, of the outsides of the fibres.

In fig.2 the overflow valve 10 is shown on a larger scale. In the embodiment shown here it consists of a prop ring 13 cast into the end wall 4, against which rests with a sealing bulge 15 an elastic U-shaped sealing ring 14. The sealing bulge 15 is shown in fig.2 in its original size, but in practice, in assembled condition, it will be pressed almost completely into the sealing ring 14 itself.

The ring 14 is slipped onto a fixed rigid flange 16 on the inside of the outer cover 6. This fixed flange 16 is provided with ports or openings 17 which are partly covered on one side by the flange 18 of the U-shaped sealing ring 14, and wholly covered on their other side by the other flange 19 of this ring. This other flange is thus longer and so elastic that it is turned outwards when it is subjected on its inside to a suitably chosen maximum pressure. In this manner any exposure of the blood to haemolysis is prevented.

## EMBODIMENT II

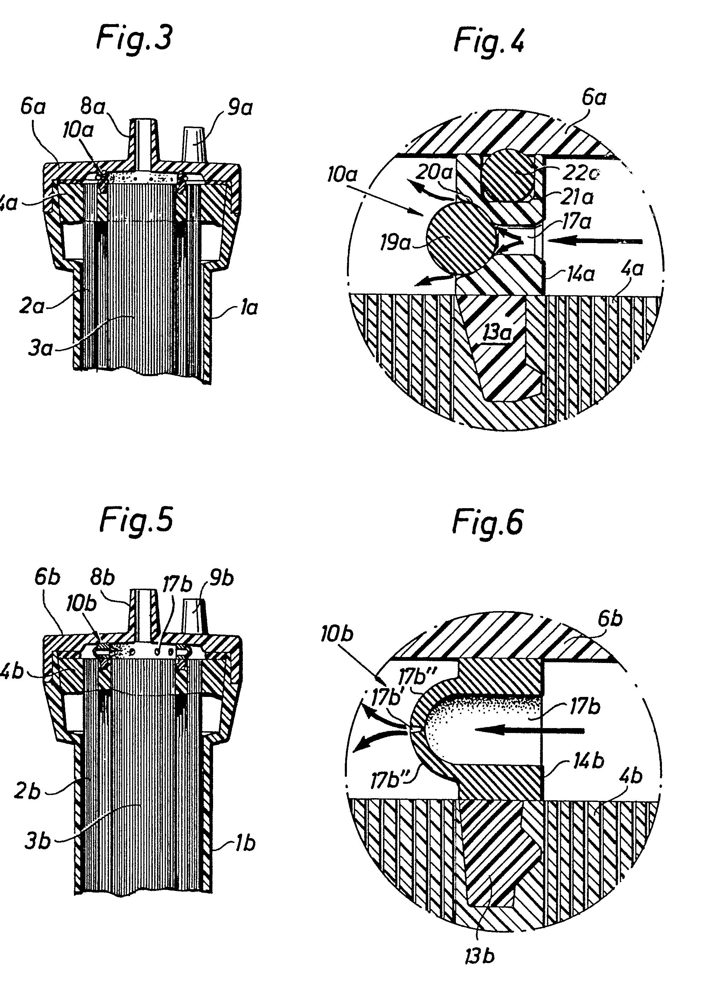

In figures 3 and 4 an alternative embodiment is shown. As it corresponds in principle with that according to figures 1 and 2, the same reference figures have been used but with the addition of the letter a. Thus the casing is designated 1a and the concentrically arranged fibre bundles 2a and 3a. In the same manner the end wall into which these fibres are cast is designated 4a, and the outer cover arranged on the outside has the numeral 6a. This outer cover is provided with an inlet 8a and an outlet 9a for the liquid filtered and an overflow valve 10a arranged in between.

The overflow valve 10a is shown on a larger scale in figure 4. It consists of a prop ring 13a cast into the end wall 4a, to which is glued, or attached in some other suitable manner, a fixed ring 14a. The ring 14a is provided with radial ports or windows 17a, the outer openings of which are covered by an elastic ring 19a. This ring 19a is arranged in a peripheral groove 20a which runs around the outside of the ring 14a. Moreover, the ring 14a is provided with an outer groove 21a with a sealing ring 22a arranged therein. This ring 22a seals against the adjoining outer cover 6a. The design according to figures 3 and 4 functions in the same manner as that according to figures 1 and 2, that is to say at a certain maximum pressure on the inside of the ring 14a, the ports 17a are opened owing to an expansion of the ring 19a.

## EMBODIMENT III

In figures 5 and 6 an alternative embodiment is shown which in principle corresponds to that described above. For this reason, the same reference designations have been used, but with the addition of the letter b. Thus a casing is designated 1 and the concentric fibre bundles enclosed therein 2b and 3b. These fibre bundles are cast into the end wall 4b on both sides of a pressure-controlled overflow valve 10b. The numerals 8b and 9b designate an inlet and an outlet respectively in the cover 6b.

The overflow valve 10b is shown on a larger scale in fig.6. As in the overflow valves described above,it comprises a prop ring 13b which is cast into the end wall 4b. An elastic sealing ring 14b rests against the prop ring which is provided with a number of radial ports or openings 17b which in the innermost part open out into an openable slit 17b'. The elastic ring 14b is adapted so as to be squeezed between the prop ring 13b and the outer cover 6b. The function is the same as for the designs described above. At a certain maximum pressure on the inside of the ring 14b, the lips 17b" open, so that the slit 17b' widens and allows the blood or any other liquid treated to pass through.

EMBODIMENT IV

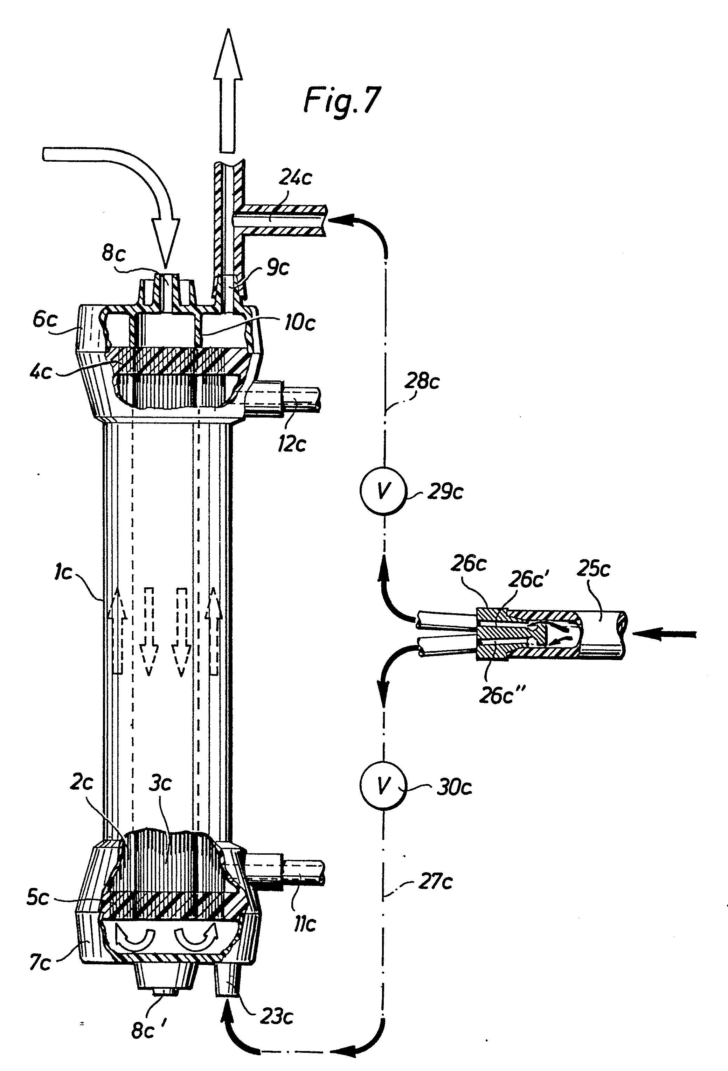

. In figures 7,8 and 9 an alternative embodiment is shown, supplemented by means for the supply of replacement liquid. The design corresponds in principle to that described above and has been given, therefore, the same reference designations, but with the addition of the letter c. Thus the casing is designated 1c and the fibres 2c and 3c respectively. In the same manner the end walls are designated 4c and 5c respectively and the outer covers 6c and 7c respectively. Numeral 8c designates the inlet for the blood and 9c its outlet. Numeral 10c designates the overflow valve which in this case is considered to consist of a single thin flange attached to the cover 6c which yields elastically, if the pressure at the inlet 8c is too high. Numeral 8c' refers to a normally plugged up inlet corresponding to inlet 8c. This inlet 8c' is meant to facilitate possible scavenging of the fibres in connection with the manufacture of the device. Numeral 11c designates an outlet for the filtrate and 12c a further corresponding inlet or outlet, which may be plugged up and which has the same function, during the manufacture of the device, as the inlet 8c'.

The design in accordance with figure 7 differs from those described above primarily by an inlet 23c for the supply of replacement liquid between the two chambers formed by the fibres 2c and 3c respectively. Furthermore, the construction is provided with a further inlet 24c for the supply of replacement liquid downstream of the chamber formed by the fibres 2c. The replacement liquid is supplied via a flexible tube 25c with together with a coupling 26c forms a distributing valve. From this valve the replacement liquid is passed via the lines 27c and 28c to the inlets 23c and 24c respectively. The liquid may pass the valves 29c and 30c respectively. Either or both these valves can be controllable so that appropriate quantities should be supplied to the particular inlet. The idea in principle is,however, that the distributing valve 25c-26c should be self-regulating. To achieve this, the ducts 26c' and 26c" are given appropriate dimensions and the elasticity in the flexible tube 25c is chosen so that the inlet area is automatically increased for whichever. duct 26c' or 26c" has the greatest back pressure.

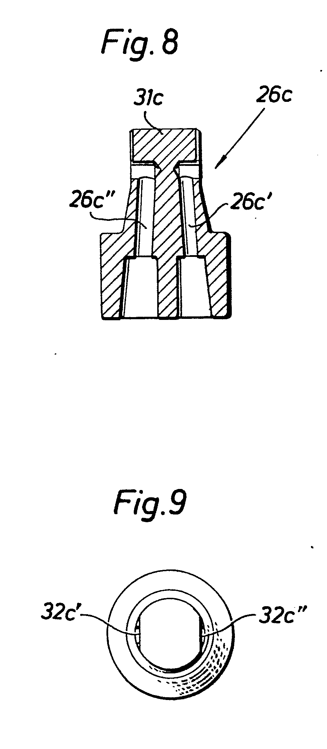

In the figures 8 and 9 the coupling 26c is shown on a larger scale. As can be seen, the spigot 31c of the coupling is provided with two bevellings 32c' and 32c" at the places where the openings of the ducts 26c' and 26" are. Hence when the flexible tube 25c is slipped over the spigot 31c a certain slight flow will take place via these bevellings. If an increase in pressure occurs in either line 27c or line 28c, an expansion of the flexible tube 25c will be obtained opposite the corresponding bevelling, so that the sectional area of flow increases, which makes possible a substantially unchanged flow.

## INDUSTRIAL APPLICABILITY

It is evident from what has been said in the foregoing that the invention is intended primarily to be used for the manufacture of membrane filtering devices for haemofiltration and plasmafers and similar medical applications.However, it will be clear to those versed in the art, that the invention can also be applied to other cases where a filtration is desired at a relatively high pressure which,however,has to be maximized. Naturally, the invention is not limited merely to the embodiments described above,but may be varied within the scope of the following claims.See also patent application........81.05851-3 submitted at the same time,which is aimed at a further development of the method for the supply of replacement liquid.

CLAIMS

1. A filtering device comprising an inlet (8) and an outlet (9) for the liquid filtered and an outlet (11) for the filtrate, the filtration being arranged to take place in two chambers (2,3) connected in series, which consist at least partly of membrane material through which the filtration is taking place, c h a r a c - t e r i z e d by a pressure-controlled overflow valve (10) arranged between the inlet (8) and the outlet (9) for the liquid filtered.

2. A filtering device in accordance with claim 1, c h a r a c t e r - i z e d in that both chambers (2,3) are arranged in one and the same casing (1) and that each is formed of a number of interior spaces of thin-walled fibres (2,3).

3. A filtering device in accordance with claim 2, the fibres (2,3) of the two chambers being cast into two end walls (4,5) of a cylindrical casing (1) which, outside these end walls (4,5) has outer covers(6,7),bearing the said inlet (8) and outlet (9) for the liquid fil- tered ,c h a r a c t e r i z e d in that the said inlet (8) and outlet (9) are arranged in one (6) of the outer covers (6,7) separated by a seal (10) arranged between the adjoining end wall and the cover, which constitutes the said pressure-controlled overflow valve (10).

4. A filtering device in accordance with claim 3, c h a r a c t e r - ized in that the seal (10) is ring-shaped and is arranged substantially concentrically on the inside of the outer cover (6) pressing against the outside of the adjoining end wall (4).

5. A filtering device in accordance with claim 4, c h a r a c t e r - i z e d in that a prop ring (13) is cast into the end wall (4)opposite the seal (10).

6. A filtering device in accordance with claim 4, c h a r a c t e r - i z e d in that the seal (10) consists of an elastic ring (14) which is slipped onto a rigid flange (16) fixed on the inside of the outer cover (6).

7. A filtering device in accordance with claim 6, c h a r a c t e r - i z e d in that the rigid flange (16) is interrupted by one or more ports (17) which are only partly covered on their one side by the elastic ring (14) which, wholly but resiliently, covers the other side of the ports.

**0076421**

8. A filtering device in accordance with claim 7, c h a r a c t e r - i z e d in that the elastic ring (14) is of a U-shaped cross-section with one leg (19) longer than the other (18).

9. A filtering device in accordance with claim 4 or 5, c h a r a c - t e r i z e d in that the seal is formed by a fixed ring (14a) arranged between the outer cover (6) and the adjoining end wall (4a), which is provided with one or more radial ports (17a), the outside of which is covered by an elastic ring (19a) slipped on outside the fixed ring (14a).

10. A filtering device in accordance with claim 9, c h a r a c t e r - i z e d in that the elastic ring (19a) is arranged in a peripheral groove (20a) in the fixed ring (14a).

11. A filtering device in accordance with claim 9 or 10, c h a r a c - t e r i z e d by an elastic sealing ring (21a) arranged between the outer cover (6a) and the fixed ring (14a).

12. A filtering device in accordance with anyone of claims 9-11, c h a r a c t e r i z e d in that the fixed rigid ring (14a) is glued, or is attached in some other manner, to the adjoining end wall (4a) or the prop ring (13a) cast into it.

13. A filtering device in accordance with claim 4 or 5, c h a r a c - t e r i z e d in that the seal (10b) is formed by an elastic ring (14b) arranged between the outer cover and the adjoining end wall, which comprises thinner wall portions (17b",17b") which tightly adhere to one another at lower pressure, but which are adapted so as to be separated at higher pressure.

1/4

Fig.1

Fig.2

0076421

## Fig.3

## Fig.4

## Fig.5

## Fig.6

Fig.7

Fig. 8

Fig.9